# EUROPEAN PATENT APPLICATION

(11) **EP 2 684 563 A1**
(43) Date of publication of application: **15.01.2014**
(21) Application number: 11860309.1
(22) Date of filing: 04.11.2011
(51) Int. Cl.: A61K 31/075, A61K 31/045, A61K 31/21, A61P 31/04

(54) **ANTIBACTERIAL OR PRESERVING COMPOSITION CONTAINING 3-BUTOXY-1,2-PROPANEDIOL**

(30) Priority: 10.03.2011 KR 20110021414; 29.07.2011 KR 20110076180
(71) Applicant: LG Household & Health Care Ltd, Jongno-gu, Seoul 110-783 (KR)
(72) Inventor: GU, Min-Ji, Daejeon 305-340 (KR); CHOI, Jung-Jin, Daejeon 305-343 (KR); JEONG, Gug-In, Daejeon 305-343 (KR); CHANG, Sug-Youn, Seoul 150-993 (KR)
(74) Representative: Icosa
(86) International application number: PCT/KR2011/008388
(87) International publication number: WO 2012/121469

(57) **Abstract**

The present invention relates to an antibacterial and/or preserving composition which contains 3-butoxy-1,2-propanediol as a preserving agent. The antibacterial or preserving composition according to the present invention has an excellent resistance to microorganisms and has an excellent moisturizing effect, and hardly causes skin troubles.

## Description

### TECHNICAL FIELD

The present invention relates to an antibacterial or preserving composition containing a certain derivative of glycerine, which has excellent antibacterial and antiseptic properties. Also, the present invention relates to a method of improving an antibacterial or preserving effect of products (preferably, cosmetics and goods for human use) by using or adding a certain derivative of glycerine.

### BACKGROUND ART

Various products, including cosmetics and goods for human use, contain various materials such as humectants, oils, surfactants, inorganic particles, water-soluble organic polymers and flavoring agents, which become the conditions that are suitable for microbial growth. Accordingly, in order to prevent such a product from being decomposed due to microorganisms and from being deteriorated by eliminating microorganisms with time, it is general to use chemical antiseptics. As such a chemical antiseptic, parabens, imidazolinyl ureas, phenoxyethanol, p-oxybenzoic acid esters and benzalkonium chloride have been conventionally used.

However, as new additives such as proteins, gums, vitamins and medicinal plants are added to products recently, various changes according to the action of microorganisms are induced. Also, as the effect of chemical antiseptics weakens, the amount thereof increases. Such increases in the amount of chemical antiseptics cause skin stimulation, allergy symptoms and are considered harmful to the human body.

Generally, various products including cosmetics and goods for human use should have good enough stability so to not decompose by microbial growth, phase separation and color change. However, anions present in cosmetic compositions, e.g., hydroxide (OH⁻), carbonate (CO₃²⁻) and phosphate (PO₄³⁻) ions are subject to reaction with metallic cations present in water, e.g., Fe³⁺ and Ca²⁺ to produce an insoluble precipitate or cause discoloration. In the case of a flavoring agent having an aldehyde or ketone structure, an oxidation reaction tends to be facilitated by a trace amount of cations of metals such as Fe, Cu and Mn. Owing to such unstability due to cations, cosmetic compositions generally comprise a chelating agent with the purpose of improving color stability and preventing discoloration, precipitation and flavoring agents' oxidation. As the chelating agent for cations, ethylene diamine acetic acid (EDTA), diethylenetriamine pentaacetic acid (DTPA) and N-hyrdoxyethyl-ethylenediamine triacetic acid (HEEDTA) have been conventionally used. The anionic compound of the chelating agent is bonded to metallic cations to form a strong ring structure. That is, the chelating agent makes metallic cations be neutral, or form a strong bond with the metallic cations to produce a new anionic compound, thereby controlling or inactivating the metallic cations and eventually improving the stability of a formulation. However, the use of only a chelating agent is insufficient to prevent the decomposition by microbial growth which is another stability factor.

Accordingly, there is a continuous need to develop a new composition capable of preventing decomposition by microbial growth even though a chemical antiseptic is contained in a small amount or none at all.

### DISCLOSURE

### Technical Problem

The present invention is designed to solve the above problems, and therefore it is an object of the present invention to provide an antibacterial or preserving composition which can exhibit excellent antibacterial or antiseptic effects with little to no chemical antiseptic, provide a good moisturizing effect without stickiness, have superior formulation stability and minimize skin troubles such as skin stimulation and allergy symptoms.

That is, the present invention provides an antibacterial or preserving use of a glycerine derivative having a distinctive structure, and such a glycerine derivative exhibits excellent antibacterial and antiseptic effects, provides good moisturizing effect without stickiness, superiorly maintains formulation stability and hardly causes skin troubles such as skin stimulation and allergy symptoms.

Accordingly, the present invention also provides a method of improving an antibacterial or preserving effect of products (preferably, cosmetics and goods for human use) by using or adding the glycerine derivative having a distinctive structure.

### Technical Solution

In order to achieve the objects as mentioned above, in accordance with one aspect of the present invention, there is provided an antibacterial and/or preserving composition, containing 3-butoxy-1,2-propanediol of formula (I):

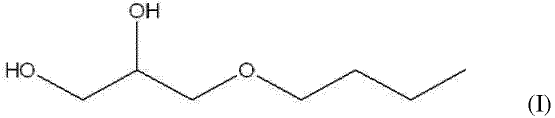

That is, the present invention provides an antibacterial or antiseptic use of 3-butoxy-1,2-propanediol of formula (I). Such 3-butoxy-1,2-propanediol has advantages which exhibits excellent antibacterial and antiseptic effects, provides a good moisturizing effect without stickiness, superiorly maintains formulation stability and hardly causes skin troubles such as skin stimulation and allergy symptoms.

Also, the present invention provides a method of improving an antibacterial or preserving effect of products (preferably, cosmetics and goods for human use) by using or adding such 3-butoxy-1,2-propanediol of formula (I).

In order to solve the problems of the prior arts, the present inventors have endeavored to develop a substance having superior antibacterial or preserving effect by testing various ingredients, and found that among the tested ingredients, such 3-butoxy-1,2-propanediol of formula (I) achieved an antibacterial or preserving effect, minimized the amount of chemical antiseptics, which hardly caused any skin trouble, as well as achieving a moisturizing effect without any stickiness.

Particularly, the antibacterial or preserving ingredient and composition according to the present invention can exhibit a synergetic effect when used together with conventional chemical antiseptics, thereby providing more excellent antibacterial or preserving effect. The antibacterial or preserving composition according to the present invention contains 3-butoxy-1,2-propanediol as a preservative to exhibit excellent antibacterial or preserving effect even though a chemical antiseptic is used in very small amounts. Accordingly, unlike conventional cosmetic compositions using an excess amount of chemical antiseptics to cause skin troubles such as skin stimulation and allergy symptoms, the antibacterial and/or preserving composition according to the present invention hardly causes such a skin trouble. As the chemical antiseptic, polyol such as 3-hexyloxy-1,2-propanediol, 1,2-hexanediol, octanediol, paraben, phenoxyethanol and a mixture thereof may be used.

In the present invention, the amount of 3-butoxy-1,2-propanediol is preferably 0.01 to 40 wt%, more preferably 0.5 to 5 wt%, based on the total weight of the composition. If the amount of 3-butoxy-1,2-propanediol is less than 0.01 wt%, the antibacterial and preserving effects are insufficient. If the amount of 3-butoxy-1,2-propanediol is higher than 40 wt%, a poor feeling of use due to stickiness may be caused.

Preferably, the antibacterial and/or preserving composition of the present invention further comprises a chelating agent for metallic cations to prevent discoloration, precipitation and flavoring agents' oxidation, thereby improving formulation stability. Such a chelating agent can provide a synergetic effect when it is used together with 3-butoxy-1,2-propanediol according to the present invention, and eventually can provide more improved antibacterial and preserving effects.

Examples of the chelating agent which may be used in the present invention include sodium diphosphate, sodium triphosphate, 1-hydroxyethane-1,1-diphosphoric acid, nitrilotrimethylenephosphoric acid, nitrilotriacetic acid, ethylenediaminetetraacetate, 1,2,3,4-cyclopentanetetracarboxylic acid, citric acid, *O*-carboxylmethyl tartronic acid, *O-*carboxylmethyloxy succinic acid and a mixture thereof. Among these, ethylenediaminetetraacetate (e.g., ethylenediaminetetraacetate-2Na, -3Na, -4Na, etc.) are preferred in terms of the object of the present invention.

In the antibacterial and/or preserving composition according to the present invention, the chelating agent may be preferably used in an amount of 0.01 to 2 wt% based on the total weight of the antibacterial or preserving composition. If the amount of the chelating agent is less than 0.01 wt%, formulation stability and antibacterial and preserving effects are insufficient. If the amount of the chelating agent is higher than 2 wt%, effects relative to cost are unsatisfied.

As mentioned above, the present invention is based on several effectiveness of 3-butoxy-1,2-propanediol. That is, the present invention provides several uses of 3-butoxy-1,2-propanediol as the ingredient of cosmetics.

Specifically, the present invention provides an antibacterial or preserving composition, characterized by containing 3-butoxy-1,2-propanediol of formula (I) to improve stability. In the present invention, 3-butoxy-1,2-propanediol being the ingredient of cosmetics is colorless and transparent liquid, and has good dissolvability and emulsion-stabilizing ability as compared with conventional humectants to substantially improve the stability of compositions. Such a stabilizing effect by the 3-butoxy-1,2-propanediol of the present invention is not based on only the theoretical assumption. That is, the 3-butoxy-1,2-propanediol according to the present invention may be used for improving the stability of various formulation including cosmetics compositions in the form of oil-in-water or water-in-oil, emulsions, microemulsions, skins, lotions, creams, foundations, essences, gels, packs, foam cleansers, softeners, astringents, mascaras, eye liners, shampoos, conditioners, soaps, body cleansers, hair conditioners, and hair tonics, but the present invention is not limited thereto.

Accordingly, the present invention provides a method of improving the stability of a cosmetic composition, characterized by adding 3-butoxy-1,2-propanediol to the cosmetic composition.

Preferably, the present invention provides an antibacterial or preserving composition in the form of oil-in-water or water-in-oil, which comprises 1 to 60 wt% of a water-based ingredient, 1 to 10 wt% of a humectant, 1 to 5 wt% of a surfactant, 1 to 30 wt% of an oil and the remaining water.

In the antibacterial or preserving composition having improved stability according to the present invention, the water-based ingredient may be any one which is soluble in water and used in cosmetics without deteriorating the object of the present invention. For example, water-soluble polymers such as guar gum, xanthan gum, soduim chondroitin lactate, sodium hyaluronate, Arabia gum, sodium alginate, collagenan, methylcellulose, hydroxymethylcellulose, carboxylvinylpolymer, polyvinylalcohol, polyvinylpyrrolidone, alkylated carboxylvinylpolymer, sodium polyacrylate; salts such as sodium chloride, magnesium chloride, sodium lactate; extracts of plants such as ginger, Phellodendron, Berberine, lithospermum, birch, loquat, ginseng, iris, graph, lily, cnidium officinale, aloe vera, cucumber, lemon, lavender, rose; and a mixture thereof may be used.

In the antibacterial or preserving composition having improved stability according to the present invention, the humectant may include 3-butoxy-1,2-propanediol according to the present invention and any other humectant which may be used without deteriorating the object of the present invention. Examples of such other humectant include water-soluble substances, e.g., glycols such as propyleneglycol, 1,3-butyleneglycol, dipropyleneglycol and polyethyleneglycol glycol; glycerols such as glycerine, diglycerine and polyglycerine; sugars such as sorbitol, mannitol, sucrose, starch sugar, lactitol; hyaluronic acid; betaine, but it is preferred that only 3-butoxy-1,2-propanediol of formula (I) is used as the humectant in terms of the object of the present invention. Such a humectant enhances the moisturizing effect of skin and lowers the polarity of water phase, from which waxes having a lower polarity, higher alcohols and surfactants can interact with the water phase to assist the formation of a stable emulsion.

In the antibacterial or preserving composition having improved stability according to the present invention, the surfactant may be nonionic, anionic, cationic or amphoteric surfactants or a mixture thereof.

The anionic surfactant is not limited if it may be used in conventional cosmetic compositions, and examples thereof include soaps made of fatty acids (such as sodium laurate and sodium palmitate); higher alcohol sulfuric acid esters (such as sodium lauryl sulfate and potassium lauryl sulfate); alkyl ether sulfuric acid esters (such as POE-triethanolamine lauryl sulfate, POE-sodium lauryl sulfate); N-acyl sarcosinates (such as sodium lauroyl sarcosinate); higher fatty acid amide sulfonates (such as sodium N-myristoyl-N-methyl taurate, sodium methyl taurate of a fatty acid from palm oil and sodium lauryl methyl taurate); phosphoric acid esters (such as POE-sodium oleyl ether phosphate and POE-stearyl ether phosphate); sulfosuccinates (such as sodium di-2-ethylhexylsulfo succinate, sodium monolauroyl monoethanol amide polyoxyethylene sulfosuccinate, and sodium lauroyl polypropylene glycol sulfosuccinate); alkyl benzene sulfonates (such as linear sodium decylbenzene sulfonate, linear triethanolamine decylbenzene sulfonate, and linear decylbenzene sulfonic acid); higher fatty acid ester sulfates (such as sodium glycerine sulfate of hardened palm oil); N-acyl glutamates (such as monosodium N-lauroly glutamate, disodium N-stearoyl glutamate, and monosodium N-myristoyl-L-glutamate); sulfated oils (such as rot oil); POE-alkyl ether carboxylic acids; POE-alkyl allyl ether carboxylate; α-olefin sulfates; higher fatty acid ester sulfates; secondary alcohol ester sulfate; fatty acid alkylol amide ester sulfates; sodium lauroyl monoethanol amide succinate; N-palmitoyl aspartic acid ditriethanolamine; sodium casein; and a mixture thereof, but is not limited thereto.

The nonionic surfactant may be lipophilic or hydrophilic and is not limited if it may be used in conventional cosmetic compositions. Examples of the lipophilic nonionic surfactant include sorbitan fatty acid esters (such as sorbitan monooleate, sorbitan monoisostearate, sorbitan monolaurate, sorbitan monopalmitate, sorbitan monostearate, sorbitan sesqueoleate, sorbitan trioleate, penta-2-ethylhexyl diglycerolsorbitan, and tetra-2-ethylhexyl acid diglycerolsorbitan); glycerine polyglycerine fatty acids (such as mono-cotton seed oil fatty acid glycerine, monoerucic acid glycerine, sesquioleic acid glycerine, monostearic acid glycerine, α,α'-oleic acid pyroglutamic acid glycerine, and monostearic acid glycerine maleic acid); propyleneglycol fatty acid esters (such as monostearic acid propyleneglycol); hardened caster oil derivatives; and glycerinealkylether. Examples of the hydrophilic nonionic surfactant include POE-sorbitan fatty acid esters (such as POE-sorbitan monooleate, POE-sorbitan monostearate, and POE-sorbitan tetraoleate); POE-sorbitol fatty acid esters (such as POE-sorbitol monolaurate, POE-sorbitol monooleate, POE-sorbitol pentaoleate, and POE-sorbitol monostearate); POE-glycerine fatty acid esters (such as POE-glycerine monostearate, POE-glycerine monoisostearate, and POE-glycerine triisostearate); POE-fatty acid esters (such as POE-distearate, POE-monodioleate, and distearic acid ethyleneglycol); POE-alkyl ethers (such as POE-lauryl ether, POE-oleyl ether, POE-stearyl ether, POE- behenyl ether, POE-2- octyldodecyl ether, and POE- cholestanol ether); Pulronic type surfactants (such as Pulronic); POE·POP-alkyl ethers (such as POE·POP-cetyl ether, POE·POP-2-decyl tetradecylether, POE·POP-monobutylether, POE·POP-hydrogenated lanolin, and POE·POP-glycerine ether); tetra POE·tetra POP-ethylenediamine condensates (such as Tetronic); POE-caster oil hardened caster oil derivatives (such as POE-caster oil, POE-hardened caster oil, POE-hardened caster oil monoisostearate, POE-hardened caster oil triisostearate, POE-hardened caster oil mono pyroglutamic acid glycerine monoisostearic acid diester, and POE-hardened caster oil maleic acid); POE beeswax lanolin derivatives (such as POE-sorbitol beeswax); alkanol amide (such as palm oil fatty acid diethanol amide, lauric acid monoethanol amide, fatty acid isopropanol amide); POE-propyleneglycol fatty acid ester; POE-alkylamine; POE-fatty acid amide; sucrose fatty acid ester; alkyl ethoxy dimethyl amineoxide; trioleic acid; and a mixture thereof, but are not limited thereto.

The cationic surfactant is not limited if it may be used in conventional cosmetic compositions, and examples thereof include alkyltrimethylammonium salts (such as stearyltrimethylammonium chloride, and lauryltrimethylammonium chloride); alkylpyridimium salts (such as cetylpyridium chloride); distearyldimethylammonium chloride; dialkyldimethylammonium salts; poly(N,N-dimethyl-3,5-methylenepiperidinium chloride); alkyl quaternary ammonium salts; alkyl dimethylbenzylammonium salts; alkyl isoquinolinium salts; dialkyl morpholin salts; POE-alkylamine; alkylamine salts; polyamine fatty acid derivatives; amyl alcohol fatty acid derivatives; benzalkonium chloride; benzethonium chloride; and a mixture thereof, but are not limited thereto.

The amphoteric surfactants is not limited if it may be used in conventional cosmetic compositions, and examples thereof include imidazoline-based amphoteric surfactants (such as sodium 2-undecyl-N,N,N-(hydroxyethylcarboxymethyl)-2-imidazoline, 2-cocoyl-2-imidazolinium hydroxide, and 1-carboxyethyloxy-2-sodium); betaine-based surfactants (such as 2-heptadecyl-N-carboxymethyl-N-hydroxyethylimidazolinium betaine, lauryl dimethylamino acetic acid betaine, alkylbetaine, amidebetaine and sulfobetaine).

Preferred surfactants are polyoxyethylene cetyl ether, polyoxyethylene oleyl ether, polyoxyethylene stearyl ether, polyoxyethylene nonylphenyl ether, polyoxyethylene lauryl ether, polyoxyethylene hexyldecyl ether, polyoxyethylene isostearyl ether, polyoxyethylene octyldodecyl ether, polyoxyethylene behenyl ether, polyoxyethylene cholesteryl ether, polyoxyethylene hardened caster oil, sorbitan ester, mono-fatty acid glycerine, tri-fatty acid glycerine, polyglycerine fatty acid ester, isostearic acid polyoxyethylene glycerine, tri-isostearic acid polyoxyethylene glycerine, monostearic acid polyoxyethylene glycerine, distearic acid polyoxyethylene glyceryl, tristearic acid polyoxyethylene glyceryl, but are not limited thereto.

In the antibacterial or preserving composition having improved stability according to the present invention, the oil is not limited if it may be used in conventional cosmetic compositions. Specifically, hydrocarbon-based oils such as squalene and mineral oil; ester-based oils such as caprilic/capric triglyceride, neopentyl glycol dicaprate, 2-octyldodecylmyristate, isopropylmyristate, isocetylethylhexanoate, pentaerythrityl tetraethylhexanoate, butylenes glycol dicaprilate caprate, hexyllaurate, distearylmaleate, cetyl 2-ethylhexanoate, octyldodecanol, and glyceryl triethylhexanoate; vegetable oils such as olive oil, avocado oil, jojoba oil, and macadamia oil; animal oils such as lanolin; silicone oils such as dimethyl polysiloxane, methylphenyl polysiloxane, decamethyl cyclopentasiloxane, methyl trimethicone, phenyl trimethicone, cyclomethicone, and dimethicone may be used alone or as a mixture thereof. The oil may act as a factor which dissolves solid components present in waxes and higher alcohols, and affects the adhesive property and spreadability of emulsions. Accordingly, the oil may affect the feeling of use and stability of emulsions depending on its kinds and amounts.

Also, the antibacterial or preserving composition having improved stability according to the present invention may further comprise higher alcohols, waxes, flavoring agents, and coloring agents within the range without deteriorating the object of the present invention, and such an additional ingredient may be selected from those conventionally used in the art.

The higher alcohols are a solid component which largely affects the viscosity of cosmetic compositions, and examples thereof include straight alcohols (such as lauryl alcohol, cetyl lcohol, stearyl alcohol, behenyl alcohol, myristyl alcohol, oleyl alcohol, and cetostearyl alcohol); and branched alcohols (such as monostearyl glycerine ether (batyl alcohol), 2-decyl-tetradecanol, lanolin alcohol, cholesterol, phytosterol, hexyldodecanol, isostearylalcohol, and octyldodecanol). The higher alcohols increase the viscosity of emulsions to inhibit agglomeration of oil particles, thereby improving formulation stability.

The waxes are a solid component maintaining the appearance of a water-in-oil or oil-in-water form, and examples thereof include Candelilla wax, ceresin, carnauba wax, paraffin, wooden wax, spermaceti wax, bee wax, cotton wax, bayberry wax, white wax, montan wax, rice bran wax, lanolin, sugar cane wax, jojoba wax, and microcrystalline wax. Such a wax provides gloss to the surface of creams, forms a protective layer on the skin for skin protection, and provides a heavy feeling of use.

### Advantageous Effects

The present invention uses 3-butoxy-1,2-propanediol as a preserving agent for an antibacterial and/or preserving composition to provide excellent resistance to microorganisms as well as excellent moisturizing effect, and hardly causes skin troubles.

### BEST MODE

Hereinafter, various preferred examples of the present invention will be described in detail for better understanding. However, the examples of the present invention may be modified in various ways, and they should not be interpreted as limiting the scope of the invention. The examples of the present invention are just for better understanding of the invention to persons having ordinary skill in the art.

### <Examples 1-3 and Comparative Examples 1-2>

The ingredients and amounts listed in Table 1 were used according to a conventional method to prepare antibacterial or preserving compositions in the form of emulsion.

**Table 1**

| Ingredients (wt%) | Example 1 | Example 2 | Example 3 | Com. Example 1 | Com. Example 2 |
|---|---|---|---|---|---|
| Distilled water | to 100 | to 100 | to 100 | to 100 | to 100 |
| 3-Butoxy-1,2 propanediol | 1 | 2 | 3 | - | - |
| Glycerine | 3 | 3 | 3 | 3 | 3 |
| Wax | 3 | 3 | 3 | 3 | 3 |
| Butter | 2 | 2 | 2 | 2 | 2 |
| Higher fatty acid | 3 | 3 | 3 | 3 | 3 |
| Polystearate | 3 | 3 | 3 | 3 | 3 |
| Xanthan gum | 0.03 | 0.03 | 0.03 | 0.03 | 0.03 |
| Flavoring agent | q. s. | q. s. | q. s. | q. s. | q.s. |
| Methyl paraben | - | - | - | 0.2 | - |
| Ethyl paraben | - | - | - | 0.1 | - |
| Propyl paraben | - | - | - | 0.1 | - |

### <Test Example 1>

The compositions of Examples 1-3 and Comparative Examples 1-2 were evaluated for their antibacterial and antiseptic effects. Each sample was contaminated with bacteria or fungi, and then a change in the number of bacteria or fungi inoculated was checked. The check was conducted for a maximum of 28 days, and the days in which the number of bacteria or fungi starts to be in the range of 20 CFU/g or less was recorded with the results shown in Table 2. The test was specifically conducted as follows.

First, each cosmetic formulation was provided in an amount of 40g, to which a mixed lysate of *Staphylococcus aureus* (ATCC 6538), *Pseudomonas aeruginosa* (ATCC 15522) and *Escherichia coli* (ATCC 10536) was added so that the total bacteria became 10⁶ numbers per 1g of sample thereof. Each sample was left for 4 weeks. After inoculation, a change in the number of the bacteria was checked for 1, 7, 14, 21 and 28 days from the inoculation.

Another test using fungi was conducted according to the same procedures as the above test using bacteria, except that fungi, *Aspergillus niger* (ATCC 9642) and *Candida albicans* (ATCC 10231) were used instead of bacteria.

**Table 2**

| Test subject | Examples | | | Comparative Examples | |
|---|---|---|---|---|---|
| | 1 | 2 | 3 | 1 | 2 |
| Bacteria | 14 days or more | 7 days | 1 day | 14 days | 28 days or more |
| Fungi | 14 days or more | 7 days | 1 day | 14 days | 25 days or more |

As shown in Table 2, in the case of Comparative Example 2, since no preservative or a chemical antiseptic was used, no antibacterial and antiseptic effect were exhibited, while Comparative Example 1 using a chemical antiseptic exhibited antibacterial and antiseptic effects, so that the number of bacteria or fungi was in the range of 20 CFU/g or less after 14 days from the inoculation.

Meanwhile, in Example 1 of the present invention, despite not using any chemical antiseptic, the number of bacteria or fungi was in the range of 20 CFU/g or less after 14 days from the inoculation. Thus, Example 1 exhibited antibacterial and antiseptic effects similar to Comparative Example 1 using a chemical antiseptic. Particularly, in Examples 2 and 3 using 3-butoxy-1,2-propanediol in an amount of 2 wt% or more, the number of bacteria or fungi was rapidly reduced, so that the number of bacteria or fungi was in the range of 20 CFU/g or less after 7 days and 1 day from the inoculation, respectively. These antibacterial and antiseptic effects are superior as compared with Comparative Example 1.

### <Test Example 2>

In order to evaluate whether the compositions of Example 2 and Comparative Example 1 cause skin stimulation, a primary stimulation test for a human body was conducted through a human patch test. The test was conducted on 50 subjects consisting of healthy adult males and females, according to CTFA guidelines disclosed in the Document [The Cosmetic, Toility and Fragrance Association. Inc. Washington, D.C., 20036.19941]. Specifically, 15 ml of a sample solution was placed in a test chamber (Finn Chamber), and held in place on the back of the subjects (test area) with an adhesive tape. The patch test was conducted for 24 hours, and then the patch was removed. After 4 hours from the removal, skin reactions of the test area was observed and rated based on the following indication standards. The results thereof are shown in Table 3.

### <Indication Standards>

- : no redness or particulars
+/- : slightly red over other places
+ : substantially red over other places
++ : severely red over other places
Degree of stimulation = [{1 (number of (+/-))} + {2 (number of (+))} + {3 (number of (++))}] / Total number of subjects

**Table 3**

| Sample | Number of Subjects | Indication | | | | Stimulation |
|---|---|---|---|---|---|---|
| | | ++ | + | +/- | - | |
| Example 2 | 50 | 0 | 0 | 2 | 48 | 0.04 |
| Com. Example 1 | 50 | 0 | 2 | 6 | 42 | 0.20 |

As shown in Table 3, Example 2 using 3-butoxy-1,2-propanediol as a preservative exhibited substantially reduced skin stimulation, as compared with Comparative Example 1 using a chemical antiseptic.

### <Examples 2, 4-8 and Comparative Examples 3-7>

The ingredients and amounts listed in Tables 4 and 5 were used according to a conventional method to prepare antibacterial or preserving compositions in the form of emulsion.

**Table 4**

| Ingredients (wt%) | Example 2 | Example 4 | Example 5 | Example 6 | Example 7 | Example 8 |
|---|---|---|---|---|---|---|
| Distilled water | to 100 | to 100 | to 100 | to 100 | to 100 | to 100 |
| 3-Butoxy-1,2 propanediol | 2 | 1 | 1 | 1 | 1 | 1 |
| 1,2-Hexanediol | - | 1 | - | - | - | - |
| Octanediol | - | - | 0.2 | - | - | - |
| 3-Hexyloxy-1,2 propanediol | - | - | - | - | 0.2 | - |
| Phenoxyethanol | - | - | - | - | - | 0.5 |
| Glycerine | 3 | 3 | 3 | 3 | 3 | 3 |
| Wax | 3 | 3 | 3 | 3 | 3 | 3 |
| Butter | 2 | 2 | 2 | 2 | 2 | 2 |
| Higher fatty acid | 3 | 3 | 3 | 3 | 3 | 3 |
| Polystearate | 3 | 3 | 3 | 3 | 3 | 3 |
| Xanthan gum | 0.03 | 0.03 | 0.03 | 0.03 | 0.03 | 0.03 |
| Flavoring agent | q.s. | q. s. | q.s. | q. s. | q.s. | q.s. |
| Methyl paraben | - | - | - | 0.1 | - | - |
| Ethyl paraben | - | - | - | 0.05 | - | - |
| Propyl paraben | - | - | - | 0.05 | - | - |

**Table 5**

| Ingredients (wt%) | Com. Example 3 | Com. Example 4 | Com. Example 5 | Com. Example 6 | Com. Example 7 |
|---|---|---|---|---|---|
| Distilled water | to 100 | to 100 | to 100 | to 100 | to 100 |
| 3-Butoxy-1,2 propanediol | - | - | - | - | - |
| 1,2-Hexanediol | 2 | - | - | - | - |
| Octanediol | - | 0.4 | - | - | - |
| 3-Hexyloxy-1,2 propanediol | - | - | - | 0.4 | - |
| Phenoxyethanol | - | - | - | - | 0.5 |
| Glycerine | 3 | 3 | 3 | 3 | 3 |
| Wax | 3 | 3 | 3 | 3 | 3 |
| Butter | 2 | 2 | 2 | 2 | 2 |
| Higher fatty acid | 3 | 3 | 3 | 3 | 3 |
| Polystearate | 3 | 3 | 3 | 3 | 3 |
| Xanthan gum | 0.03 | 0.03 | 0.03 | 0.03 | 0.03 |
| Flavoring agent | q.s. | q.s. | q.s. | q.s. | q.s. |
| Methyl paraben | - | | 0.2 | - | - |
| Ethyl paraben | - | | 0.1 | - | - |
| Propyl paraben | - | | 0.1 | - | - |

### <Test Example 3>

In order to test an improvement in antibacterial and antiseptic effects according to the synergetic effect of 3-butoxy-1,2-propanediol and a chemical antiseptic, each sample of the compositions of Examples 2, 4-8 and Comparative Examples 3-7 was contaminated with bacteria or fungi, and then a change in the number of bacteria or fungi inoculated was checked. The check was conducted for a maximum of 28 days, and the days in which the number of bacteria or fungi starts to be in the range of 20 CFU/g or less was recorded with the results thereof shown in Table 6. The specific procedures are the same as Test 1.

**Table 6**

| Test subject | Example | | | | | | Comparative Example | | | | |
|---|---|---|---|---|---|---|---|---|---|---|---|
| | 2 | 4 | 5 | 6 | 7 | 8 | 3 | 4 | 5 | 6 | 7 |
| Bacteria | 7 days | 1 day | 4 days | 5 days | 4 days | 7 days | 10 days | 10 days | 14 days | 14 days | 14 days |
| Fungi | 7 days | 1 day | 4 days | 5 days | 4 days | 7 days | 10 days | 10 days | 14 days | 14 days | 14 days |

As shown in Table 6, Comparative Examples 3 to 7 using a chemical antiseptic exhibited the number of bacteria or fungi in the range of 20 CFU/g or less after 10 days, whereas Examples 2 and 4 to 8 using 3-butoxy-1,2-propanediol exhibited the number of bacteria or fungi in the range of 20 CFU/g or less after 7 days, thereby exhibiting a more superior antibacterial and antiseptic effects as compared with the Comparative Examples.

Particularly, in Examples 4 to 7 using 3-butoxy-1,2-propanediol together with a trace amount of a chemical antiseptic, a synergetic effect was achieved to exhibit substantially improved antibacterial and antiseptic effects as compared to Example 2 using only 3-butoxy-1,2-propanediol.

### <Test Example 4>

In order to evaluate whether the compositions of Examples 5-6 and Comparative Examples 4-5 cause skin stimulation, a primary stimulation test for a human body was conducted by the same method as Test 2, and the results thereof are shown in Table 7.

**Table 7**

| Sample | Number of subjects | Indication | | | | Degree of stimulation |
|---|---|---|---|---|---|---|
| | | ++ | + | +/- | - | |
| Example 5 | 50 | 0 | 2 | 4 | 44 | 0.16 |
| Example 6 | 50 | 0 | 1 | 3 | 46 | 0.10 |
| Com. Example 4 | 50 | 1 | 2 | 10 | 37 | 0.34 |
| Com. Example 5 | 50 | 0 | 2 | 8 | 40 | 0.24 |

As shown in Table 7, Examples 5 and 6 using 3-butoxy-1,2-propanediol as a preservative and a trace amount of a chemical antiseptic exhibited substantially reduced skin stimulation, as compared with Comparative Examples 4 and 5 using a relatively excess amount of the chemical antiseptic.

### <Examples 2, 9-10 and Comparative Example 8>

The ingredients and amounts listed in Table 8 were used according to a conventional method to prepare antibacterial or preserving compositions in the form of emulsion.

**Table 8**

| Ingredients (wt%) | Example 2 | Example 9 | Example 10 | Com. Example 8 |
|---|---|---|---|---|
| Distilled water | to 100 | to 100 | to 100 | to 100 |
| 3-Butoxy-1,2 propanediol | 2 | 2 | 2 | - |
| Glycerine | 3 | 3 | 3 | 3 |
| Ethylene diaminetetraacetate-4Na | - | 0.1 | - | 0.1 |
| Nitrilotriacetic acid | - | - | 0.1 | - |
| Wax | 3 | 3 | 3 | 3 |
| Butter | 2 | 2 | 2 | 2 |
| Higher fatty acid | 3 | 3 | 3 | 3 |
| Polystearate | 3 | 3 | 3 | 3 |
| Xanthan gum | 0.03 | 0.03 | 0.03 | 0.03 |
| Flavoring agent | q.s. | q.s. | q.s. | q.s. |
| Methyl paraben | - | - | - | 0.2 |
| Ethyl paraben | - | - | - | 0.1 |
| Propyl paraben | - | - | - | 0.1 |

### <Test Example 5>

In order to test an improvement in antibacterial and antiseptic effects according to the synergetic effect of 3-butoxy-1,2-propanediol and a chelating agent, each sample of the compositions of Examples 2, 9-10 and Comparative Example 8 was contaminated with bacteria or fungi, and then a change in the number of bacteria or fungi inoculated was checked. The check was conducted for a maximum of 28 days, and the days in which the number of bacteria or fungi starts to be in the range of 20 CFU/g or less was recorded with the results thereof shown in Table 9. The specific procedures are the same as Test 1.

**Table 9**

| Test subject | Examples | | | Com. Example 8 |
|---|---|---|---|---|
| | 2 | 9 | 10 | |
| Bacteria | 7 days | 1 day | 4 days | 14 days |
| Fungi | 7 days | 1 day | 4 days | 14 days |

As shown in Table 9, Comparative Example 8 using a chemical antiseptic exhibited the number of bacteria or fungi in the range of 20 CFU/g or less after 14 days, whereas Examples 2, 9 and 10 using 3-butoxy-1,2-propanediol exhibited the number of bacteria or fungi in the range of 20 CFU/g or less after 7 days, thereby exhibiting more superior antibacterial and antiseptic effects as compared with the Comparative Example.

Particularly, in Examples 9 and 10 using 3-butoxy-1,2-propanediol together with a chelating agent, a synergetic effect was achieved to exhibit substantially improved antibacterial and antiseptic effects as compared to Example 2 using only 3-butoxy-1,2-propanediol.

### <Test Example 6>

In order to evaluate whether the compositions of Example 9 and Comparative Example 8 cause skin stimulation, a primary stimulation test for a human body was conducted by the same method as Test 2, and the results thereof are shown in Table 10.

**Table 10**

| Sample | Number of subjects | Indication | | | | Degree of stimulation |
|---|---|---|---|---|---|---|
| | | ++ | + | +/- | - | |
| Example 9 | 50 | 0 | 1 | 2 | 47 | 0.08 |
| Comparative Example 8 | 50 | 0 | 2 | 8 | 40 | 0.24 |

As shown in Table 10, Example 9 using 3-butoxy-1,2-propanediol as a preservative exhibited substantially reduced skin stimulation, as compared with Comparative Example 8 using a chemical antiseptic.

## Claims

1. An antibacterial or preserving composition, containing 3-butoxy-1,2-propanediol of formula (I):

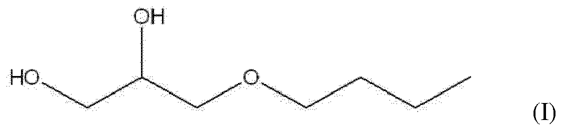

2. The antibacterial or preserving composition of claim **1,** wherein 3-butoxy-1,2-propanediol is present in an amount of 0.01 to 40 wt% based on the total weight of the composition.

3. The antibacterial or preserving composition of claim **1,** which further comprises at least one chemical antiseptic selected from the group consisting of 3-hexyloxy-1,2-propanediol, 1,2-hexanediol, octanediol, paraben, phenoxyethanol and a mixture thereof.

4. The antibacterial or preserving composition of claim **1,** which further comprises a chelating agent to improve the stability of the composition.

5. The antibacterial or preserving composition of claim **4,** wherein the chelating agent is selected from the group consisting of sodium diphosphate, sodium triphosphate, 1-hydroxyethane-1,1-diphosphoric acid, nitrilotrimethylenephosphoric acid, nitrilotriacetic acid, ethylenediaminetetraacetate, 1,2,3,4-cyclopentanetetracarboxylic acid, citric acid, *O*-carboxylmethyl tartronic acid, *O*-carboxylmethyloxy succinic acid and a mixture therof.

6. The antibacterial or preserving composition of claim **4,** wherein the chelating agent is ethylenediaminetetraacetate.

7. The antibacterial or preserving composition of claim **4,** wherein the chelating agent is present in an amount of 0.01 to 2 wt% based on the total weight of the composition.

8. A method of improving an antibacterial or preserving effect of a composition by adding 3-butoxy-1,2-propanediol of formula (I):

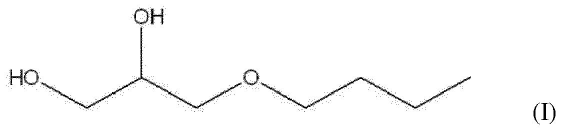

9. A use of 3-butoxy-1,2-propanediol of formula (I) as an antibacterial agent or preservative:
